# EUROPEAN PATENT SPECIFICATION

(11) **EP 1 952 145 B1**
(45) Date of publication and mention of the grant of the patent: **21.04.2010**
(21) Application number: 06819567.6
(22) Date of filing: 16.11.2006
(51) Int. Cl.: G01N 33/50, C12Q 1/68

(54) **A METHOD FOR SPECIFIC DETECTION OF LEGIONELLA PNEUMOPHILA**
VERFAHREN ZUM SPEZIFISCHEN NACHWEIS VON LEGIONELLA PNEUMOPHILA
PROCEDE DE DETECTION SPECIFIQUE DE LA LEGIONELLA PNEUMOPHILA

(30) Priority: 18.11.2005 EP 05110953
(43) Date of publication of application: 06.08.2008
(73) Proprietor: UNIVERSITAT DE GIRONA, 17003 Girona (ES)
(72) Inventor: CALVÓ, Laia, E-17003 Girona (ES); GARCÍA-GIL, Jesús, E-17003 Girona (ES)
(74) Representative: ZBM Patents
(86) International application number: PCT/EP2006/068592
(87) International publication number: WO 2007/057437

(56) References cited:
- EP-A- 0 943 691
- EP-A- 1 176 213
- WO-A-00/10584
- WO-A-01/42791
- US-B1- 6 194 145
- ENGLEBERG N C ET AL: "DNA SEQUENCE OF MIP, A LEGIONELLA PNEUMOPHILA GENE ASSOCIATED WITH MACROPHAGE INFECTIVITY" INFECTION AND IMMUNITY, AMERICAN SOCIETY FOR MICROBIOLOGY. WASHINGTON, US, vol. 57, no. 4, April 1989 (1989-04), pages 1263-1270, XP002066527 ISSN: 0019-9567
- AURELL HELENA ET AL: "Rapid detection and enumeration of Legionella pneumophila in hot water systems by solid-phase cytometry" APPLIED AND ENVIRONMENTAL MICROBIOLOGY, WASHINGTON,DC, US, vol. 70, no. 3, March 2004 (2004-03), pages 1651-1657, XP002304543 ISSN: 0099-2240

### SEQUENCE LISTING

<110> Universitat de Girona
<120> A method for specific detection of Legionella pneumophila
<130> P639PC00
<150> EP05110953.6.
   <151> 2005-11-18
<160> 8
<170> PatentIn version 3.3
<210> 1
   <211> 282
   <212> DNA
   <213> Legionella pneumophila
<220>
   <221> CDS
   <222> (1)..(282)
<300>
   <308> GenBank/AE017354 REGION: 585004..585285
   <309> 2004-09-24
   <313> (1)..(282)
<400> 1
   atg aca aca atc agt aat gat gca gga gat acc act gct tca cta gcc      48
   Met Thr Thr Ile Ser Asn Asp Ala Gly Asp Thr Thr Ala Ser Leu Ala
   1               5                   10                  15
   gaa agc gtg act caa tcc gta caa aaa tat ttt tca gag ctt aag gga      96
   Glu Ser Val Thr Gln Ser Val Gln Lys Tyr Phe Ser Glu Leu Lys Gly
               20                  25                  30
   act gat cct gtt gac tta tac cag ttt gta ctt gaa gaa att gag acc     144
   Thr Asp Pro Val Asp Leu Tyr Gln Phe Val Leu Glu Glu Ile Glu Thr
           35                  40                  45
   cct cta ttt cgt gca gta atg gaa cat tgc aag tat aat cag tcc cgc     192
   Pro Leu Phe Arg Ala Val Met Glu His Cys Lys Tyr Asn Gln Ser Arg
       50                  55                  60
   gct gca atc atg ctg gga att agt cgt gga act tta aga act aaa tta     240
   Ala Ala Ile Met Leu Gly Ile Ser Arg Gly Thr Leu Arg Thr Lys Leu
   65                  70                  75                  80
   aga cgt tat ttt gat gat aaa tac gtt ggt aca agg gat taa             282
   Arg Arg Tyr Phe Asp Asp Lys Tyr Val Gly Thr Arg Asp
                   85                  90
<210> 2
   <211> 93
   <212> PRT
   <213> Legionella pneumophila
<400> 2
   Met Thr Thr Ile Ser Asn Asp Ala Gly Asp Thr Thr Ala Ser Leu Ala
   1               5                   10                  15
   Glu Ser Val Thr Gln Ser Val Gln Lys Tyr Phe Ser Glu Leu Lys Gly
               20                  25                  30
   Thr Asp Pro Val Asp Leu Tyr Gln Phe Val Leu Glu Glu Ile Glu Thr
           35                  40                  45
   Pro Leu Phe Arg Ala Val Met Glu His Cys Lys Tyr Asn Gln Ser Arg
       50                  55                  60
   Ala Ala Ile Met Leu Gly Ile Ser Arg Gly Thr Leu Arg Thr Lys Leu
   65                  70                  75                  80
   Arg Arg Tyr Phe Asp Asp Lys Tyr Val Gly Thr Arg Asp
                   85                  90
<210> 3
   <211> 297
   <212> DNA
   <213> Legionella pneumophila
<220>
   <221> CDS
   <222> (1)..(297)
<300>
   <308> GenBank/AE017354 REGION: complement(1516880..1517176)
   <309> 2004-09-24
   <313> (1)..(297)
<400> 3
   atg aat gtc att gat aca caa gct aca act caa tcc act aaa caa gaa        48
   Met Asn Val Ile Asp Thr Gln Ala Thr Thr Gln Ser Thr Lys Gln Glu
   1               5                   10                  15
   caa ggt tta cag gat tta gtt tac agt ttg gtt acc cgc ttt ctt gct        96
   Gln Gly Leu Gln Asp Leu Val Tyr Ser Leu Val Thr Arg Phe Leu Ala
               20                  25                  30
   gaa aat aaa tcc aaa tca att aat gat ctc tat gat atg atc ctg tca       144
   Glu Asn Lys Ser Lys Ser Ile Asn Asp Leu Tyr Asp Met Ile Leu Ser
           35                  40                  45
   gaa gtt gag cca cct cta tta caa gct gta atg gaa aaa cgt cgt gga       192
   Glu Val Glu Pro Pro Leu Leu Gln Ala Val Met Glu Lys Arg Arg Gly
       50                  55                  60
   aat caa tta caa gca gcc aag atg ttg ggt att agt cgc ggt acg atc       240
   Asn Gln Leu Gln Ala Ala Lys Met Leu Gly Ile Ser Arg Gly Thr Ile
   65                  70                  75                  80
   aga aag aaa tta caa aga tat ttc ggt acc aaa tat ttt cgc tta act       288
   Arg Lys Lys Leu Gln Arg Tyr Phe Gly Thr Lys Tyr Phe Arg Leu Thr
                   85                  90                  95
   gaa gag taa                                                           297
   Glu Glu
<210> 4
   <211> 98
   <212> PRT
   <213> Legionella pneumophila
<400> 4
   Met Asn Val Ile Asp Thr Gln Ala Thr Thr Gln Ser Thr Lys Gln Glu
   1               5                   10                  15
   Gln Gly Leu Gln Asp Leu Val Tyr Ser Leu Val Thr Arg Phe Leu Ala
               20                  25                  30
   Glu Asn Lys Ser Lys Ser Ile Asn Asp Leu Tyr Asp Met Ile Leu Ser
           35                  40                  45
   Glu Val Glu Pro Pro Leu Leu Gln Ala Val Met Glu Lys Arg Arg Gly
       50                  55                  60
   Asn Gln Leu Gln Ala Ala Lys Met Leu Gly Ile Ser Arg Gly Thr Ile
   65                  70                  75                  80
   Arg Lys Lys Leu Gln Arg Tyr Phe Gly Thr Lys Tyr Phe Arg Leu Thr
                   85                  90                  95
   Glu Glu
<210> 5
   <211> 288
   <212> DNA
   <213> Legionella pneumophila
<220>
   <221> CDS
   <222> (1)..(288)
<300>
   <308> GenBank/AE017354 REGION: complement(1945064..1945351)
   <309> 2004-09-24
   <313> (1)..(288)
<400> 5
   atg agt gca gtt atg caa caa gtg aca caa ggt aac gat gcc ttg tca      48
   Met Ser Ala Val Met Gln Gln Val Thr Gln Gly Asn Asp Ala Leu Ser
   1               5                   10                  15
   cat caa gtg att agt gca gtt aaa ggt tat tta aca agt gtc ggt agt      96
   His Gln Val Ile Ser Ala Val Lys Gly Tyr Leu Thr Ser Val Gly Ser
               20                  25                  30
   aaa gat gct aat tta aac tta tat caa ttg att gtt gag gaa gta gaa      144
   Lys Asp Ala Asn Leu Asn Leu Tyr Gln Leu Ile Val Glu Glu Val Glu
           35                  40                  45
   gcc cct tta ttc cgg act gtt atg gaa tta act cgc tat aat caa tca      192
   Ala Pro Leu Phe Arg Thr Val Met Glu Leu Thr Arg Tyr Asn Gln Ser
       50                  55                  60
   aaa gct gct cga gtg ctt ggt gta agt cgt ggt act ttg cgc act aaa      240
   Lys Ala Ala Arg Val Leu Gly Val Ser Arg Gly Thr Leu Arg Thr Lys
   65                  70                  75                  80
   tta aag cgt tat ttt gat gat gaa ttc att ggt act cgt gat ttt taa      288
   Leu Lys Arg Tyr Phe Asp Asp Glu Phe Ile Gly Thr Arg Asp Phe
                   85                  90                  95
<210> 6
   <211> 95
   <212> PRT
   <213> Legionella pneumophila
<400> 6
   Met Ser Ala Val Met Gln Gln Val Thr Gln Gly Asn Asp Ala Leu Ser
   1               5                   10                  15
   His Gln Val Ile Ser Ala Val Lys Gly Tyr Leu Thr Ser Val Gly Ser
               20                  25                  30
   Lys Asp Ala Asn Leu Asn Leu Tyr Gln Leu Ile Val Glu Glu Val Glu
           35                  40                  45
   Ala Pro Leu Phe Arg Thr Val Met Glu Leu Thr Arg Tyr Asn Gln Ser
       50                  55                  60
   Lys Ala Ala Arg Val Leu Gly Val Ser Arg Gly Thr Leu Arg Thr Lys
   65                  70                  75                  80
   Leu Lys Arg Tyr Phe Asp Asp Glu Phe Ile Gly Thr Arg Asp Phe
                   85                  90                  95
<210> 7
   <211> 21
   <212> DNA
   <213> Artificial sequence
<220>
   <223> PCR primer
<400> 7
   cactagccga aagcgtgact c 21
<210> 8
   <211> 26
   <212> DNA
   <213> Artificial sequence
<220>
   <223> PCR primer
<400> 8
   atgttccatt actgcacgaa atagag 26

## Description

### FIELD OF INVENTION

The present invention relates to a method for specific detection of the presence of *Legionella pneumophila* in a sample that is suspected to contain *L. pneumophila* and which further comprises one or more other microorganism(s).

### BACKGROUND

*Legionella pneumophila* is a gram-negative, rod-shaped bacterium that is causative agent for a high percentage of both community-acquired and nosocomial pneumonias. When legionellosis occurs, it can be fatal if the diagnosis and treatment are not promptly established, particularly in elderly and immuno-compromised patients. Therefore, rapid detection and diagnostic methods are needed to improve the outcome of infected patients.
Within the genus of *Legionella* are a number of other not *L. pneumophila* species which are not giving significant virulence problems for humans.

Detection of *Legionella* is nowadays being performed both environmentally and clinically.

In the environment, it is typically found in a number of aquatic environments including those more directly related with human activities such as cooling towers and domestic drinking water distribution systems. More recently, the presence of this bacterium has been demonstrated in groundwater.

In clinical samples, *Legionella* can be directly obtained from sputum, broncho-alveolar lavage or traqueal aspiration. In advanced legionellosis, it also can be cultured from blood samples.

Recently, the molecular detection by means of PCR-based techniques has become a common procedure for the rapid identification of *Legionella.* Both conventional and modern real-time PCR protocols have been implemented, targeting a number of genes containing unique, signature sequences.

PCR-based methods described to date target a number of phylogenetic and functional genes include oligonucleotides specifically targeting regions of the ribosomal operon such as the 23S-5S (Herpers, B. L., et al, (2003), J. Clin. Microbiol., 41, 4815-4816) spacer or the 16S rRNA (Lisby et al, (1994) Eur J Clin Microbiol Infect Dis, 13, 225-231).

However, functional genes involved in virulence and infectivity are currently the markers of choice for most PCR procedures. The most widely used genes to date are *mip* (macrophage infectivity potentiator) and *dotA* (defect in organelle trafficking). The gene *mip* was actually the first molecular (DNA) target for the PCR detection of Legionella and encodes for a 24-kDa protein (*Mip*) a virulence factor consisting of a surface protein which facilitates *L. pneumophila* to parasitize human macrophages and to cause pneumonia in experimental animals. *Mip* contains specific sequences usable to distinguish Legionella species among them (Mahbubani, et al, (1990), Mol Cell Probes, 4, 175-187). The gene *dotA* (defect in organelle trafficking) encodes for a protein that regulates trafficking of the L. pneumophila phagosome (Roy, et al, (1998), Mol Microbiol, 28, 663-674). US5935782 describes use of *frgA* and *hbp* genes for detection of *L. pneumophila.* The *frgA* and *hbp* genes are functionally related to *mip* and can therefore also be characterized as genes involved in virulence and infectivity.

However, all these genes related to virulence and pathogenicity factors (e.g. *mip* and *dotA*) are likely to be quiescent during normal environmental life of this microorganism, and therefore cannot be used for determination of viable bacteria in environmental samples.

### SUMMARY OF THE INVENTION

The problem to be solved by the present invention is to provide a method for specifically detecting *Legionella pneumophila,* wherein the method provides for the possibility of determining if there is viable *Legionella pneumophila* in e.g. environmental samples.

The solution is based on that the present inventors have identified that a specific group of *Legionella* genes known under the term *fis* (factor for inversion stimulation) comprise sufficient specific sequences usable to specifically detect *Legionella pneumophila* in a sample which further comprises one or more other microorganism(s) such as one or more other *Legionella* specie(s) than *L. pneumophila.*

The gene *fis* belong to the "transcription" family of genes, category K. *Fis* genes are known in different organisms such as e.g. *E. coli* and *Salmonella.* It is also known to be present in *Legionella pneumophila* (see below for further details). A "transcription" gene such as *fis* is critical for cell growth and may be termed a "house-keeping" gene. It is known to the skilled person that such "house-keeping" genes are generally quite conserved within different species of a genus. However, as said above, surprisingly the *fis* genes of *Legionella pneumophila* as described herein comprise sufficient specific sequences usable to specifically distinguish *Legionella pneumophila* from other different Legionella species. See e.g. results 2.3 of working examples herein, where it is demonstrated that *Legionella pneumophila* can be specifically distinguished from a number of other *Legionella* species and other relevant microorganisms too. The results provided in the results 2.3 section are based on conventional PCR using genomic DNA and primers oriented toward a *Legionella pneumophila fis* gene as described herein.

Further, the "house-keeping" *fis* genes as described herein have, as normal for "house-keeping" genes, a detectable expression levels during latent phases of their life cycle. Accordingly, by measuring mRNA expression levels one is capable of determining if there is viable *Legionella pneumophila* present in environmental samples. See results 2.7 of working examples herein, where this is demonstrated based on use of Reverse Transcriptase (RT) PCR.

This is a major advantage over the prior art use of virulence and pathogenicity factor genes (e.g. *mip* and *dotA*) which generally only have a significant expression level during virulence and viability can therefore only be determined in human clinical samples and not in environmental samples (e.g. in aquatic environments obtained from buildings).

Furthermore, functional genes like the ones listed in the background section above are normally subjected to strong variability, mainly because silent mutations in the third base of the codon. This means that in for example a 21-base pairs oligonucleotide, up to seven positions are in risk to be nonspecific, due to natural genetic variability of bacterial populations, which can compromise the specificity of the PCR system. For some reason, the gene *fis* as discussed herein, does not show this variability, makes it an ideal target because (i) it is a functional gene with detectable and quantitable mRNA expression, and (ii) it has a highly conserved sequence.

The whole genome sequence of *Legionella pneumophila* subsp. pneumophila str. Philadelphia 1 is described in [Chien, et al (2004), The genomic sequence of the accidental pathogen Legionella pneumophila, Science, 305, 1966-1968]. The complete genome sequence has the GenBank accession number AE017354. The herein described three *fis* genes of *Legionella pneumophila* are described in Chien, et al. The *fis* genes are:
the gene herein termed *fis1*. It has the GenBank accession number: "AE017354 REGION: 585004..585285" and the DNA sequence is shown in SEQ ID NO 1. The corresponding amino acid sequence is herein termed FIS1 and has the protein ID: AAU26638.1 and the amino acid sequence are shown in SEQ ID NO 2;
the gene herein termed *fis2.* It has the GenBank accession number: "AE017354 REGION: complement(1516880..1517176)" and the DNA sequence is shown in SEQ ID NO 3. The corresponding amino acid sequence is herein termed FIS2 and has the protein ID: AAU27452.1 and the amino acid sequence are shown in SEQ ID NO 4; and
the gene herein termed *fis3*. It has the GenBank accession number: "AE017354 REGION: complement(1945064..1945351)" and the DNA sequence is shown in SEQ ID NO 5. The corresponding amino acid sequence is herein termed FIS2 and has the protein ID: AAU27822.1 and the amino acid sequence are shown in SEQ ID NO 6.

It is known to the skilled person that there may be some relatively minor sequence differences among the similar genes within different subspecies (or serogroups) of specie of interest (here *Legionella pneumophila*). Based on common general knowledge and current available bioinformatics it is routine for the skilled person to identify such relatively minor sequence difference and determine if a gene of interest in specific subspecies is equivalent to a similar gene in another subspecies.

Accordingly, a first aspect of the invention relates to a method for specific detection of the presence of *Legionella pneumophila* in a sample that is suspected to contain *L. pneumophila* and which further comprises one or more other microorganism(s), characterized by that
(i): the sample is analyzed to identify for presence of a *Legionella pneumophila fis* (factor for inversion stimulation) gene; and
(ii) the amount of the *Legionella pneumophila fis* gene present in the sample is evaluated and if the sample comprises the *Legionella pneumophila fis* gene it is a proof for that *Legionella pneumophila* is present in the sample;
wherein the *Legionella pneumophila fis* gene is a *fis* gene selected from the group of *fis* genes consisting of:
(a) a *fis* gene comprising a DNA sequence which is at least 95% identical to the DNA sequence shown in positions 1-282 of SEQ ID NO 1 (termed "*fis1*");
   (a1) a *fis* gene that encodes a polypeptide which is at least 95% identical to the polypeptide shown in positions 1-93 of SEQ ID NO 2 (termed "FIS1");
(b) a *fis* gene comprising a DNA sequence which is at least 95% identical to the DNA sequence shown in positions 1-297 of SEQ ID NO 3 (termed "*fis2*");
   (b1) a *fis* gene that encodes a polypeptide which is at least 95% identical to the polypeptide shown in positions 1-98 of SEQ ID NO 4 (termed "FIS2");
(c) a *fis* gene comprising a DNA sequence which is at least 95% identical to the DNA sequence shown in positions 1-288 of SEQ ID NO 5 (termed "*fis3*"); and
   (c1) a *fis* gene that encodes a polypeptide which is at least 95% identical to the polypeptide shown in positions 1-95 of SEQ ID NO 6 (termed "FIS3").

The term "sample that is suspected to contain *L. pneumophila"* relates to the objective of the method of the present invention, which is to analyze if the sample comprises *L*. *pneumophila* or in a preferred embodiment viable *L. pneumophila*. Said in other words, if one is 100% sure that the sample comprises *L. pneumophila* or viable *L. pneumophila* there is no significant reason to analyze for the presence of it.

### BRIEF DESCRIPTION OF THE DRAWINGS

FIG. 1: Alignment of the three Fis protein sequences encoded in the genome of *L. pneumophila.* * indicates absolutely conserved positions
FIG. 2: Specificity test with several *Legionella* spp DNA templates. Lanes 1-8: *L pneumophila*; 9: *L gormanii*; 10: *L. longbeacheae*; 11: *L. anisa*; 12: *L. oakridgensis;* 13: *L. fairfieldensis*; 14: *L. feelei*; 15: *L. dumofii*; 16: *L. micdadei*; 17: *L. jordanis*; 18: *L. wadsworthii*; 19: *L. bozemanii*; 20-26: *L. pneumophila*; 27: *L. gormanii*; 28: *L. longbeacheae*; 29: *L. anisa*; 30: *L. oakridgensis*; 31: *L. fairfieldensis*; 32: *L. feelei*; 33: *L. dumofii*; 34: *L. micdadei*; 35: *L. jordanis*; 36: *L. wadsworthii*; 37: *L. bozemanii*; 38: Non template control.
FIG. 3: Agarose gels, showing the result of the RT + PCR assay on actively growing cells of *Legionella pneumophila.* Lanes: L, 100bp ladder; 1-3, triplicate sample; 4, Mix without RNA; 5, Mix without RT; 6, Non template control; 7, positive control.

### DETAILED DESCRIPTION OF THE INVENTION

### Sample

The sample may e.g. be a clinical sample (preferably obtained from a human) or be a so-called environmental sample. Preferably the sample is an environmental sample.

Preferably the "environmental sample" is a sample obtained from an aquatic environment. Preferably the aquatic environment is situated in a herein relevant place such as a building [preferably a domestic building (including hotels, hospitals, fitness centers, etc..)], a cooling tower, a domestic drinking water distribution system, a hot water system (e.g. pipelines), groundwater or a urban spring or other aerosol-generating water sources.

As said above the sample further comprises one or more other microorganism(s).

Examples of other microorganisms include one or more microorganism(s) selected from the group consisting of other *Legionella* species than *L. pneumophila*, *E. coli, Salmonella, Shigella, Enterobacter, Micrococcus, Bacillus, Staphylococcus, Pseudomonas, Serratia, Proteus*, *Enterococcus, Arthrobacter* and *Listeria.*

As said above, an advantage of the present invention is that *Legionella pneumophila* can be specifically distinguished from a number of other *Legionella* species.

Accordingly, in a preferred embodiment the one more other microorganism(s) comprised within the sample is one or more other *Legionella* specie(s) than *L. pneumophila.*

Examples of other *Legionella* specie(s) than *L. pneumophila* include one or more *Legionella* specie(s) selected from the group consisting of *L gormanii, L. longbeacheae, L. anisa, L. oakridgensis, L. fairfieldensis, L. feelei, L. dumofii, L. micdadei, L. jordanis, L. wadsworthii* and *L. bozemanii.*

### fis (factor for inversion stimulation) gene

As explained above, the gene *fis* belong to the "transcription" family of genes, category K. *Fis* genes are known in different organisms including *Legionella pneumophila.*

The term *fis* gene is widely known to the skilled person and based on his common general knowledge the skilled person can routinely determine whether or not a gene of interest is a *fis* gene. Examples of this are the GenBank *fis* annotations in the *fis1* to *fis2* GenBank references given above.

Below is described some relevant *fis* gene relevant information, which shall be seen as a mere illustration of common general knowledge with respect to the *fis* gene.

The gene *fis* (factor for inversion stimulation), encodes for a pleiotropic regulator of the expression of polymerases, cell-division related proteins and many ribosomal genes, and is involved in DNA replication and recombination.
The Fis protein is a member of the group of proteins known as histone-like proteins or nucleoid-associated proteins. It plays a critical role in many cellular functions ranging from the control of virulence genes in *E. coli* and *Salmonella,* to the enhancement of the expression of some genes.

Fis is a histone-like protein whose main functional characteristic is the regulation of transcription through a physical interaction with the chromosomic DNA molecule. Nevertheless, this protein can be defined in many ways, as it can be found in the literature. These alternative definitions are:
- (Positive) regulator of stable RNA operons (RNA transcription)
- Nucleoid-binding protein
- Nucleoid-associated (regulator) protein
- Histone-like protein
- (Small dimeric) DNA-bending protein
- Trans-acting protein
- DNA architectural protein

Fis is a small, basic, DNA-bending protein that has been primarily shown to both stimulate DNA inversions and activate ribosomal RNA transcription in *Escherichia coli* by interacting with the promoter. It also functions in many other reactions including phage lambda site-specific recombination, transcriptional activation of rRNA and tRNA operons repression of its own synthesis and *oriC*-directed DNA replications.

Other functions of FIS include regulation of the expression of virulence factors in enteropathogenic *E. coli* as well as *Shigella or Salmonella,* as well as enhancement of the expression of certain genes acting as a Class I activator.
FIS was first demonstrated to act by bending DNA when it was reported that this protein binds and bends the *oriC*.
Fis determines DNA topology both by regulation of topoisomerase activity and, as previously inferred, by directly reshaping DNA. FIS has been proposed to be involved in coupling cellular physiology to the topology of the bacterial cell. The protein acts by stabilizing a DNA microloop whose topology is coupled to the local topological transitions generated during the initiation of transcription. In summary, Fis modulates the topology of DNA in a growth-phase dependent manner functioning homeostatically to counteract excessive levels of negative superhelicity. Thus, this protein forms tightly bent DNA structures, or microloops, that are necessary for the optimal expression of the promoter.
The expression of the *fis* gene strongly responds to alterations in the topology of DNA in vivo, being maximal at high levels of negative supercoiling. In addition, it has been found to be strongly dependent on the growth rate in *E.* coli. This property is of interest for the determinative purpose of this patent since the gene expression can be amplified by a factor of 6 (by stimulation growth rate), which can bring to detectable levels lower densities of viable cells after the appropriate expression stimulation method has been applied.

Accordingly, in a preferred embodiment there are added *Legionella* growth nutritional compounds to the sample before analyzing the presence of mRNA in accordance with step (i) of the first aspect to stimulate growth rate of the *Legionella pneumophila.*

With respect to the first aspect of the invention is for the relevant *fis* genes said that there shall be at least 95% identity to relevant reference sequences. For all of the relevant *fis* genes of the first aspect of the invention the identity percentage is preferably at least 97.5% identity to relevant reference sequences.

In results 2.3 of working examples herein is the *fis1* gene used to specifically distinguish *Legionella pneumophila* from a number of other *Legionella* species.

Accordingly, the *Legionella pneumophila fis* gene is preferably a *fis* gene selected from a group of *fis* genes consisting of:
(a) a fis gene comprising a DNA sequence which is at least 95% identical to the DNA sequence shown in positions 1-282 of SEQ ID NO 1 (termed "*fis1*"); and
   (a1) a fis gene that encodes a polypeptide which is at least 95% identical the polypeptide shown in positions 1-93 of SEQ ID NO 2 (termed "FIS1").

In an even more preferred embodiment, the *Legionella pneumophila fis* gene is a *fis* gene selected from a group of *fis* genes consisting of:
(a) a fis gene comprising a DNA sequence which is identical to the DNA sequence shown in positions 1-282 of SEQ ID NO 1 (termed "*fis1*"); and
   (a1) a fis gene that encodes a polypeptide which is identical the polypeptide shown in positions 1-93 of SEQ ID NO 2 (termed "FIS1").

In line of this, for the *fis2* and *fis3* genes it is preferred that they are selected from a group of *fis* genes consisting of
(b) a fis gene comprising a DNA sequence which is identical to the DNA sequence shown in positions 1-297 of SEQ ID NO 3 (termed "*fis2*");
   (b1) a fis gene that encodes a polypeptide which is identical the polypeptide shown in positions 1-98 of SEQ ID NO 4 (termed "FIS2");
(c) a fis gene comprising a DNA sequence which is identical to the DNA sequence shown in positions 1-288 of SEQ ID NO 5 (termed "*fis3*"); and
   (c1) a fis gene that encodes a polypeptide which is identical the polypeptide shown in positions 1-95 of SEQ ID NO 6 (termed "FIS3").

As said above in working examples herein is the *fis1* gene used to specifically distinguish *Legionella pneumophila* from a number of other *Legionella* species. However, based on his common general knowledge and the sequence based instructions given herein it is routine work for the skilled person to make e.g. a similar PCR based system based on the *fis2* or *fis3* genes to make an alternative system for specifically detecting *Legionella pneumophila.*

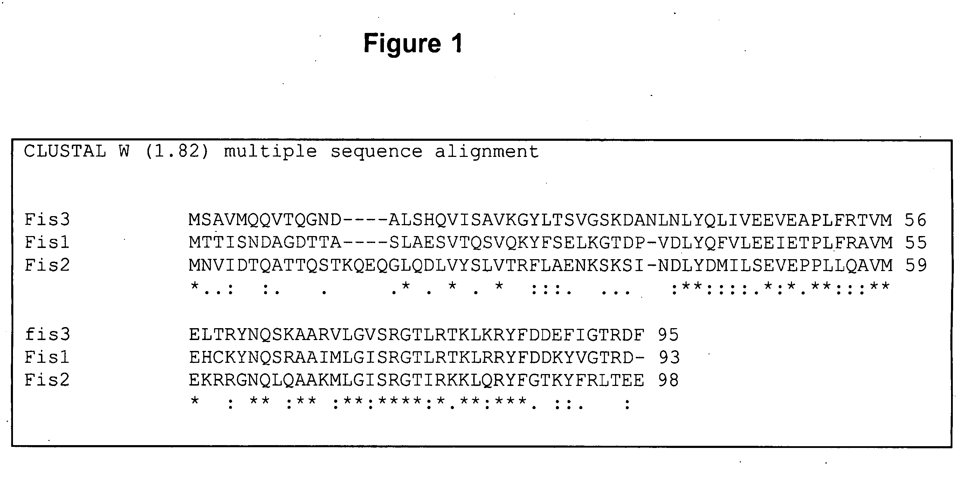

An alignment of the protein sequences of the three *fis* genes shows that the FIS1 and FIS3 are the most similar sequences, sharing a 48% homology. In turn FIS2 has a 40% similarity with FIS1 and FIS3. Further, the alignment of the three *fis* sequences reveals that homology is higher for the second half of the sequences, revealing then possible presence of critical residues for the protein function (see Figure 1). Accordingly, these second half of the sequences could be a good basis for e.g. making *Legionella pneumophila* specific PCR primers.

### Analyzing the sample to identify for presence of a Legionella pneumophila fis gene

In step (i) of the first aspect the sample is analyzed to identify for presence of a *Legionella pneumophila fis* (factor for inversion stimulation) gene.

As explained above, an advantage of using *fis* genes as described herein relates to that by measuring mRNA expression levels one is capable of determining if there is viable *Legionella pneumophila* present in e.g. environmental samples.

Accordingly, a preferred embodiment of the invention is wherein the method, as described herein, is a method for detection of viable *Legionella pneumophila* in a sample, characterized by that
(i): the sample is analyzed to identify for presence of mRNA expressed from a *Legionella pneumophila fis* (factor for inversion stimulation) gene; and
(ii) the amount of the mRNA expressed from the *Legionella pneumophila fis* gene present in the sample is evaluated and if the sample comprises the mRNA from the *Legionella pneumophila fis* gene it is a proof for that viable *Legionella pneumophila* is present in the sample.

As said above, an advantage of the present invention is that *Legionella pneumophila* can be specifically distinguished from other microorganisms (e.g. other *Legionella* species) present in the sample.

Accordingly, in a preferred embodiment the sample is analyzed by a suitable technique capable of specifically identifying the analyzed *Legionella pneumophila fis* (factor for inversion stimulation) gene or mRNA expressed from the gene and do not identify measurable amounts of *fis* gene sequences from the one or more other microorganism(s) (e.g. other *Legionella* species) further comprised within the sample.

The term "measurable amounts" should be understood as the skilled person would understand it in the present context, i.e. as an amount which does not give rise to a significant amount of what according to the art may be termed "false positives". For instance, if a PCR technique is used for the analysis then there should not be significant measurable amounts of an amplified PCR band, from other organisms, with e.g. a similar size as the "positive" amplified PCR band from *Legionella pneumophila.*

Performing the analysis as described herein may routinely be done in a number of ways. However since e.g. an environmental sample generally comprises relatively small amount of *Legionella* it is generally preferred to use an amplification technique to amplify the relevant gene sequence of mRNA expressed from this.

The art describes a number of such amplification techniques including polymerase chain reaction (PCR) or ligase chain reaction (LCR) based technology. There are also described techniques that may be said to be based on amplification under isothermal conditions, such as NASBA (nucleic acid sequence-based amplification, described in PCT Public. No. WO 91/02818) or the "Strand Displacement Amplification" method, termed SDA, which is described in US5270184.

Use of any of such amplification techniques is a routine task for the skilled person and they represent suitable examples of herein relevant amplification techniques.

Accordingly, a preferred embodiment the method, as described herein, is wherein the analysis, to identify for presence of a *Legionella pneumophila fis* (factor for inversion stimulation) gene or mRNA expressed from the *fis* gene in accordance with step (i) of the method, is done by a suitable gene amplification technique [e.g. polymerase chain reaction (PCR), ligase chain reaction (LCR), NASBA (nucleic acid sequence-based amplification) or Strand Displacement Amplification (SDA)] to amplify the relevant gene or mRNA expressed from the gene.

Further it is particular preferred wherein the amplification technique is performed in a way wherein it is capable of specifically amplifying the analyzed *Legionella pneumophila fis* (factor for inversion stimulation) gene or mRNA expressed from the gene and do not amplify measurable amounts of *fis* gene sequences from the one or more other microorganism(s) further comprised within the sample.

Preferably, the suitable gene amplification technique is PCR (preferably real-time PCR) and wherein the PCR primers are constructed in a way so the PCR primers specifically amplify the analyzed *Legionella pneumophila fis* (factor for inversion stimulation) gene or mRNA expressed from the gene and do not amplify measurable amounts of *fis* gene sequences from the one or more other microorganism(s) further comprised within the sample.

In a preferred embodiment the analysis is performed by use of a Reverse Transcriptase (RT) PCR technique, wherein there is used adequate PCR primers. In an even more preferred embodiment there is used real-time PCR technique.
As known to the skilled person RT-PCR is a technique where expressed mRNA is converted into cDNA (use of Reverse Transcriptase enzyme) and the cDNA of interest may then be PCR amplified by use of appropriate primers.

Accordingly, in a preferred embodiment the PCR technique is Reverse Transcriptase (RT) PCR and there is detected for presence of viable *Legionella pneumophila* in the sample by specifically amplify mRNA expressed from the analyzed *Legionella pneumophila fis* (factor for inversion stimulation) gene.

Preferably there is used real-time PCR combined with suitable fluorescent hybridization techniques to add sensitivity to the detection methods and e.g. considerably shortening the time per analysis.

Further, use of techniques such as PCR in addition allows bacterial load estimation in a given sample by approaching the total number through the quantization of the number of genomic copies of the targeted gene.
In this respect it is a further advantage that there is normally only one copy per genome of the *fis L. pneumophila* genes as described herein.

In a preferred embodiment, the PCR primers are constructed in a way wherein the PCR primers amplify mRNA expressed from a *Legionella pneumophila fis* gene and do not amplify *fis* gene mRNA from one or more other *Legionella* species.

As explained herein, based on his common knowledge and the information provided herein it is routine work for the skilled person to make such *Legionella pneumophila fis* gene specific primers. See e.g. working examples herein where it is done for the *fis1* gene.

In working example 1 herein is used the primers shown in SEQ ID NO 7 (termed Fis41 F) and SEQ ID NO 8 (termed Fis171R). Accordingly, in a preferred embodiment the PCR primers are selected from the group of PCR primers consisting of:
SEQ ID NO 7 (termed Fis41 F): 5'- CAC TAG CCG AAA GCG TGA CTC -3'; and
SEQ ID NO 8 (termed Fis171 R): 5' ATG TTC CAT TAC TGC ACG AAA TAG AG -3'.

### Identity of DNA sequences:

The DNA sequence identity referred to herein is determined as the degree of identity between two sequences indicating a deviation of the first sequence from the second.

At the filing date of the present invention, the National Center for Biotechnology Information (NCBI) offered at the Internet site (http://www.ncbi.nlm.nih.gov/) allows the possibility of making a standard BLAST computer sequence homology search.

BLAST program is described in [Altschul et al (1997), "Gapped BLAST and PSI-BLAST: a new generation of protein database search programs", Nucleic Acids Res. 25:3389-3402].

In the present context, a preferred computer homology search program is a "Standard nucleotide-nucleotide BLAST [blastn]" search as specified, at the filing date of the present application, at the NCBI Internet site with setting filter: Low complexity; Expect: 10, Word Size: 11.

The reference sequence is introduced into the program and the program identifies fragments of another sequence (e.g. a published sequence) together with the identity percentage to a corresponding fragment of the reference sequence.

According to the common understanding of the skilled person, when there herein is discussed an identity to a specific reference sequence to another sequence, said another sequence should have a length which is comparable to the references sequence. For instance, if the length of the reference sequence is 200 bp a comparable length of the other sequence could e.g. be from 150 -250 bp. The same applies for identity of amino acid sequences as described herein.

### Identity to amino acid sequences

Similar to the nucleotide homology analysis, in the present context, a preferred computer homology search program is a "Standard protein-protein BLAST [blastp]" search as specified, at the filing date of the present application, at the NCBI Internet site with settings Composition-based statistics: yes, filter: Low complexity; Expect: 10, Word Size: 3, Matrix: BLOSUM 62, Gap Costs: Existence 11 Extension 1.

### A separate independent aspect of the invention:

As explained above, the gene *fis* belong to the "transcription" family of genes, category K, according to COG (Cluster of Orthologous Groups) of protein functional categories annotation (Tatusov, et al (2001), Nucl. Acids Res., 29, 22-28). In *L*. *pneumophila,* this group is represented by 109 genes.

Without being limited to theory it is believed that a substantial amount of category K genes would meet the herein discussed criteria for being a good target gene to detect a viable microorganism of interest.

Accordingly, a separate independent aspect of the invention relates to a method for specific detection of the presence of viable microorganism of interest in a sample that is suspected to contain the viable microorganism of interest and which further comprises one or more other microorganism(s), characterized by that
(i): the sample is analyzed to identify for presence of mRNA expressed from a category K gene of the microorganism of interest; and
(ii) the amount of the category K gene mRNA of the microorganism of interest present in the sample is evaluated and if the sample comprises the relevant mRNA it is a proof for that viable microorganism of interest has been detected in the sample.

In a preferred embodiment the sample is analyzed by a suitable technique capable of specifically identifying mRNA expressed from a gene of a specific microorganism of interest in a sample comprising category K gene mRNA from one or more other species of the same genus as the microorganism of interest.

In a preferred embodiment the microorganism of interest is a specie of interest within a genus selected from the group consisting of *Legionella, Escherichia Shigella and Salmonella.*

In a preferred embodiment the category K gene is a category K gene selected from the group consisting of:
nusG: transcription antitermination protein NusG;
rpoB: RNA polymerase B-subunit;
rpoN: RNA polymerase sigma-54 factor (sigma-L);
rpoS: RNA polymerase sigma factor RpoS;
birA: biotin-[acetylCoA carboxylase] holoenzyme synthetase and biotin operon repressor;
mfd: Transcription-repair coupling factor;
relA: GTP pyrophosphokinase;
recG: ATP-dependent DNA helicase RecG;
hypB: hydrogenase nickel incorporation protein HypB;
greA: transcription elongation factor GreA; and
rho: transcription termination factor Rho.

All the individual preferred embodiments described above (e.g. use of PCR technology etc) with respect to the first aspect of the invention are also individual preferred individual embodiments with respect to the separate independent aspect of the invention described in this section.

### EXAMPLES

### 1. MATERIAL AND METHODS

### 1.1 Organisms used in this study and DNA extraction

Fifteen serogroups of *Legionella pneumophila* were used to test the specificity of the primers and the Taqman probe used. In addition, a total of 30 different bacterial species belonging to all major phylogenetic lineages, including 11 species of *Legionella* spp., have been used as negative specificity controls.

### 1.2 DNA extraction and quantitation

DNA from clinical specimens was extracted by using the kit NucleoSpin Blood as specified by the manufacturer (Macherei-Nagel). DNA concentration was determined by the PicoGreen™ method (Moleculsr Probes) by comparing fluorescence values with those of a calibration curve built up from a dilution series of Salmon sperm DNA (Sigma chemicals).

### 1.3 Primer Design

Partial sequences of the gene *fis1* from all serogroups of Legionella pneumophila were obtained and aligned, resulting to be identical. This fragment was then used to design two set of primers. The first one was intended to be used in conventional PCR assays for those assays requiring a presumptive (presence/absence) determination of L. pneumophila. Both forward Fis41F (5'- CAC TAG CCG AAA GCG TGA CTC -3') (SEQ ID NO 7) and reverse Fis171R (5' ATG TTC CAT TAC TGC ACG AAA TAG AG -3') (SEQ ID NO 8) primers were evaluated with the NetPrimer software (PREMIER Biosoft International, Palo Alto, California) for the formation of primer-dimer structures and hairpins.
The second set of primer was designed for the quantitative determination of L. pneumophila by real-time PCR. After introducing the consensus *fis1* sequence in the software Primer Express™ v. 2.0 (Applied Biosystems, Forster City California) optimal primers set and Taqman™ probe were obtained.

### 1.4 PCR conditions

Conventional PCR was carried out in 20 µl (total volume) reaction mixtures by using a thermal cycler (model 9600 P.E. Applied Biosystems, Foster City, CA, USA). PCR conditions were 95°C for 10 min; 40 cycles consisting of 94°C for 35 sec, 60°C for 35 sec and 72°C for 35 sec; and a final extension step consisting of 72°C for 10 min. Reaction mixtures contained 50-100 ng DNA template, 2.5 mM MgCl₂, 0.25 µM of each primer, 0.8 mM dNTP mix, and 0.5 U of TaqGold (P.E. Applied Biosystems, Forster City, CA, USA). An internal amplification control consisting of ca. 100 amplicon copies were added to a parallel reaction in order to control false negatives by ensuring that no PCR inhibition was being produced.

### 1.5 Sequencing

The PCR amplified products were sequenced in both directions with the same primers used for the PCR. DNA was sequenced as specified by the manufacturer on a 310 DNA Sequencer (Applied Biosystems, Foster City, CA, USA) using the dRhodamine Dye terminator cycle sequencing kit (Applied Biosystems, Foster City, CA, USA).

### 1.6 DNA sequence analysis

Multiple sequence alignment of partial sequences was carried out with ClustalW and was further refined manually. Phylogenetic trees were constructed by using distance, maximum likelihood and maximum parsimony methods. Minimal evolution distance trees were generated from a distance matrix obtained with the neighbor-joining algorithm with 100 bootstrap replicates using the software MEGA v.2.1 (Kumar et al. 2001). Maximum parsimony trees were also built with MEGA, with 100 bootstrap replicates while maintaining a 50% majority-rule consensus. Phylogenetic relationships were further inferred using the maximum likelihood approach with 100 bootstrap replicates. The inference was performed by Quartet Puzzling analysis feature of Treepuzzle software (Heiko et al., (1999), TREE-PUZZLE. Korbinian Strimmer and Arndt von Haeseler Heiko, A. Schmidt Theoretical Bioinformatics Deutsches Krebsforschungszentrum DKFZ, Heidelberg, Germany).

### 2. RESULTS

### 2.1 The gene fis1

The analytical system specifically targets gene *fis1,* which unlike the other markers of choice used elsewhere (*mip, dotA*) is not unique for Legionella pneumophila. It is a widespread gene among eubacterias whose functions are not related to or dependent on inducible activities such as pathogenesis. Instead, the protein encoded by the gene *fis1* is essential for sustaining cell life and viability. Both the gene and the protein sequences have been compared with those of related organisms, showing relatively high phylogenetic distances, which considerably eased the task of finding specific oligonucleotides.

### 2.2 Primer design

A degenerate primer set targeting a fragment of around 300bp of the *fis1* gene was first designed and used in a PCR with genomic DNA of the 15 serogroups of *Legionella pneumophila.* PCR products of the expected size were obtained and sequenced for the 15 serogroups.
The obtained sequences were used for designing a new non-degenerate primer set flanking a region of 130 bp of the gene *fis1*.
The non-degenerate primers were: Fis41 F and Fis171 R.
A PCR product of around 130 bp was obtained for each of the 15 serogroups of *Legionella pneumophila* tested.

### 2.3 Specificity test

PCR (the non-degenerate primers above were used) using genomic DNA of several *Legionella* spp and other bacteria from several subgroups of the Proteobacteria as template was performed. Results were positive in all the L. pneumophila tested including all serogroups from 1 to 15 (Table 1). Negative results were obtained for Legionella spp. as well as for the rest of bacterial species representing different taxa and phylogenetic lineages.

| Table 1. Bacteria used in the specificity test of the different sets of primers. | | | | |
|---|---|---|---|---|
| | | | | |
| Species | Gram | Phylogenetic affiliation | PCR | IAC |
| *Legionella pneumophila**(1 to 15) | - | γ- Proteobacteria | + | + |
| *L gormanii* | - | γ- Proteobacteria | - | + |
| *L. longbeacheae* | - | γ- Proteobacteria | - | + |
| *L. anisa* | - | γ- Proteobacteria | - | + |
| *L. oakridgensis* | - | γ- Proteobacteria | - | + |
| *L. fairfieldensis* | - | γ- Proteobacteria | - | + |
| *L. feelei* | - | γ- Proteobacteria | - | + |
| *L. dumofii* | - | γ- Proteobacteria | - | + |
| *L. micdadei* | - | γ- Proteobacteria | - | + |
| *L. jordanis* | - | γ- Proteobacteria | - | + |
| *L. wadsworthii* | - | γ- Proteobacteria | - | + |
| *L. bozemanii* | - | γ- Proteobacteria | - | + |
| *Escherichia coli* ATCC 10536 | - | γ-proteobacteria | - | + |
| *Shigella* spp. | - | γ-proteobacteria | - | + |
| *Shigella sonnei* CECT457 | - | γ-proteobacteria | - | + |
| *Salmonella* LT2 | - | γ-proteobacteria | - | + |
| *Enterobacter aerogenes* | - | γ-proteobacteria | - | + |
| *Micrococcus luteus* | + | Firmicutes | - | + |
| *Bacillus megaterium* | + | Firmicutes | - | + |
| *Staphylococcus epidermidis* | + | Firmicutes | - | + |
| *Staphylococcus aureus* | + | Firmicutes | - | + |
| *Pseudomonas fluorescens* | - | γ-proteobacteria | - | + |
| *Serratia marcescens* | - | γ-proteobacteria | - | + |
| *Pseudomonas aeruginosa* | - | γ-proteobacteria | - | + |
| *Proteus mirabilis* | - | γ-proteobacteria | - | + |
| *Bacillus subtilis* | + | Firmicutes | - | + |
| *Bacillus cereus* | + | Firmicutes | - | + |
| *Enterococcus faecalis* | + | Firmicutes | - | + |
| *Arthrobacter* VP1 | + | Firmicutes-Actinobacteria | - | + |
| *Listeria inocua* | + | Firmicutes | - | + |

| | | | | |
|---|---|---|---|---|
| *serogroups 1 to 15. IAC stands for Internal Amplification Control. A low number of template copies to control false negatives produced by inhibition. | | | | |

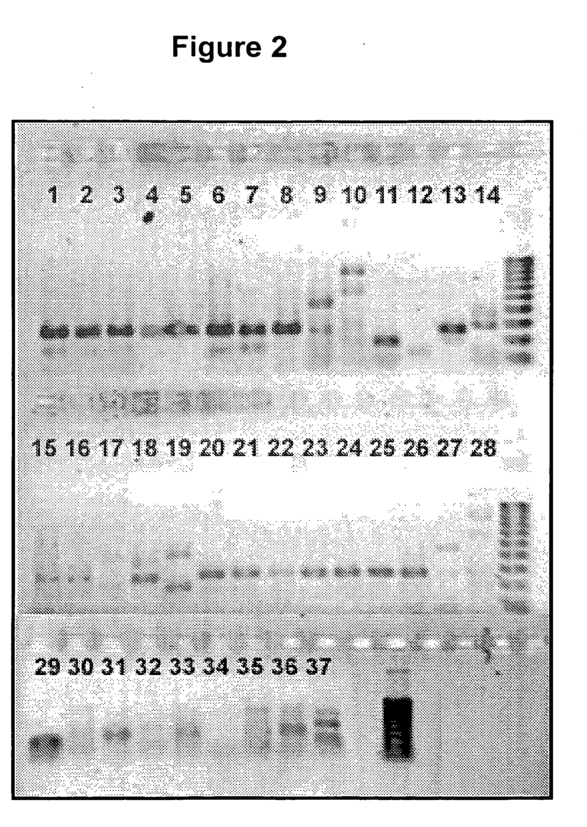

The results of the conventional PCR on different Legionella spp. using the primers of choice were positive for all tested serogroups of Legionella pneumophila and negative in the rest. This negative results was either represented by no PCR product or one or more inespecific bands of different size than expected (Figure 2).

### 2.4 Sensitivity test

Previously quantified genomic DNA from *Legionella pneumophila* SG1 was used as a target over a range of DNA concentrations in order to determine the detection limit of this method. Conventional PCR amplification was observed from as little as 600 copies of the target *fis* gene using 1 µl of the template extract.

Since the PCR reaction admits 10 µl of DNA extract as template (see below), the methods hereby presented allows to detect as low as 60 genomic copies µl-1 of DNA extract. Since the extraction method ends up with a 50 µl extract volume, we can conclude that our method is able to detect as low as 300 copies of L. pneumophila genomes per sample subjected to DNA extraction.

### 2.5 Reproducibility test

A reproducibility test was also performed. Ten replicas of a previously determined positive urine sample from a patient diagnosed with legionellosis were performed. Positive results were obtained in all reactions.

### 2.6 Maximal sample (DNA extract) load test

To evaluate the effect of using different amounts of template in the 20 µl PCR reaction, a test was also performed using 1, 5, and 10 µl of genomic DNA from clinical positive samples. Results indicate that the reaction is not inhibited even when using as much as 10 µl of purified DNA as a template.
This has important implications on the detection levels of the oligonucleotides used.

### 2.7 The gene fis1 as a marker for the detection of viable cells of Legionella pneumophila

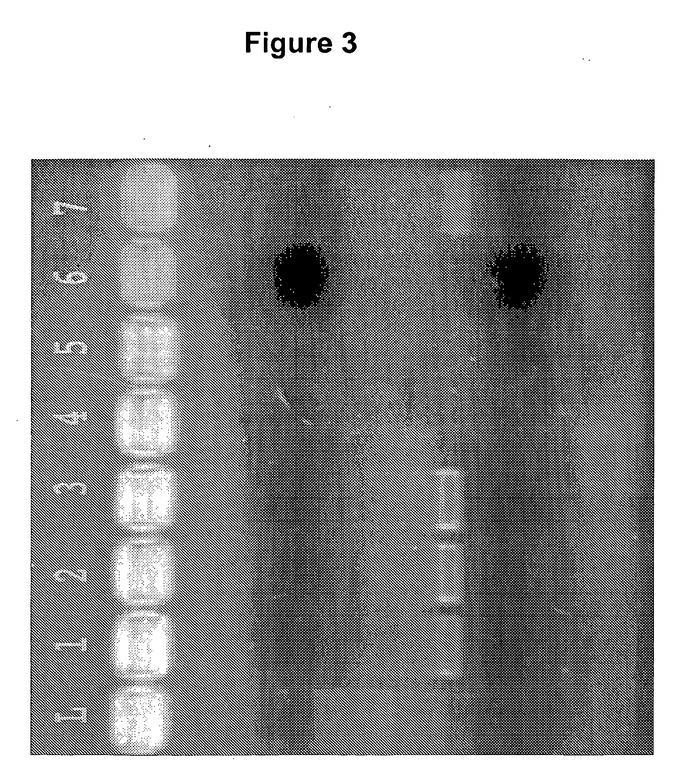

As mentioned above, the gene *fis1* is essential for maintaining the cell activity, and therefore its expression should be kept to certain limits even in dormant or latent cells, the so-called viable but non cultivable. Thus, viable Legionella pneumophila can be detected by real-time PCR after a previous Reverse Transcriptase step to backtranslate mRNA into cDNA. This was been successfully tested as shown in Figure 3.

According to the available bibliography, the expression of this gene can be dramatically increased under certain physiological conditions (e.g. addition of growth nutritional), which can be used as an amplifier when cell activity falls below the detection limit of the technique.

## Claims

1. A method for specific detection of the presence of *Legionella pneumophila* in a sample that is suspected to contain *L. pneumophila* and which further comprises one or more other microorganism(s), **characterized by** that
(i): the sample is analyzed to identify for presence of a *Legionella pneumophila fis* (factor for inversion stimulation) gene; and
(ii) the amount of the *Legionella pneumophila fis* gene present in the sample is evaluated and if the sample comprises the *Legionella pneumophila fis* gene it is a proof for that *Legionella pneumophila* is present in the sample;
wherein the *Legionella pneumophila fis* gene is a *fis* gene selected from the group of *fis* genes consisting of:
(a) a *fis* gene comprising a DNA sequence which is at least 95% identical to the DNA sequence shown in positions 1-282 of SEQ ID NO 1 (termed "*fis1*");
(a1) a *fis* gene that encodes a polypeptide which is at least 95% identical to the polypeptide shown in positions 1-93 of SEQ ID NO 2 (termed "FIS1");
(b) a *fis* gene comprising a DNA sequence which is at least 95% identical to the DNA sequence shown in positions 1-297 of SEQ ID NO 3 (termed "*fis2*");
(b1) a *fis* gene that encodes a polypeptide which is at least 95% identical to the polypeptide shown in positions 1-98 of SEQ ID NO 4 (termed "FIS2");
(c) a *fis* gene comprising a DNA sequence which is at least 95% identical to the DNA sequence shown in positions 1-288 of SEQ ID NO 5 (termed "*fis3*"); and
(c1) a *fis* gene that encodes a polypeptide which is at least 95% identical to the polypeptide shown in positions 1-95 of SEQ ID NO 6 (termed "FIS3").

2. The method of claim 1, wherein the one or more other microorganism(s) comprised within the sample is one or more microorganism(s) selected from the group consisting of *other Legionella* species than *L. pneumophila, E. coli, Salmonella, Shigella, Enterobacter, Micrococcus, Bacillus, Staphylococcus, Pseudomonas, Serratia, Proteus, Enterococcus, Arthrobacter* and *Listeria.*

3. The method of claim 1, wherein the one or more other microorganism(s) comprised within the sample is one or more other *Legionella* specie(s) than *L. pneumophila,* such as in particular one or more *Legionella* specie(s) selected from the group consisting of *L gormanii, L. longbeacheae, L. anisa, L. oakridgensis, L. fairfieldensis, L. feelei, L. dumofii, L. micdadei, L. jordanis, L. wadsworthii* and *L. bozemanii.*

4. The method of any of the preceding claims, wherein the method is a method for detection of viable *Legionella pneumophila* in a sample, **characterized by** that
(i): the sample is analyzed to identify for presence of mRNA expressed from a *Legionella pneumophila fis* (factor for inversion stimulation) gene; and
(ii) the amount of the mRNA expressed from the *Legionella pneumophila fis* gene present in the sample is evaluated and if the sample comprises the mRNA from the *Legionella pneumophila fis* gene it is a proof for that viable *Legionella pneumophila* is present in the sample.

5. The method of any of the preceding claims, wherein the sample is an environmental sample obtained from an aquatic environment, preferably wherein the aquatic environment is situated in a relevant place such as a building, a cooling tower, a domestic drinking water distribution system or groundwater.

6. The method of any of the preceding claims, wherein the *Legionella pneumophila fis* gene is a *fis* gene selected from a group of *fis* genes consisting of:
(a) a fis gene comprising a DNA sequence which is at least 95% identical to the DNA sequence shown in positions 1-282 of SEQ ID NO 1 (termed "*fis1*"); and
(a1) a fis gene that encodes a polypeptide which is at least 95% identical the polypeptide shown in positions 1-93 of SEQ ID NO 2 (termed "FIS1");
or more preferably, wherein the *Legionella pneumophila fis* gene is a *fis* gene selected from a group of *fis* genes consisting of:
(a) a fis gene comprising a DNA sequence which is identical to the DNA sequence shown in positions 1-282 of SEQ ID NO 1 (termed "*fis1*"); and
(a1) a fis gene that encodes a polypeptide which is identical the polypeptide shown in positions 1-93 of SEQ ID NO 2 (termed "FIS1").

7. The method of any of the preceding claims, wherein the analysis, to identify for the presence of a *Legionella pneumophila fis* (factor for inversion stimulation) gene or mRNA expressed from the *fis* gene in accordance with step (i) of the method, is done by a suitable gene amplification technique [e.g. polymerase chain reaction (PCR), ligase chain reaction (LCR), NASBA (nucleic acid sequence-based amplification) or Strand Displacement Amplification (SDA)] to amplify the relevant gene or mRNA expressed from the gene and wherein the amplification technique is performed in a way wherein it is capable of specifically amplifying the analyzed *Legionella pneumophila fis* (factor for inversion stimulation) gene or mRNA expressed from the gene and do not amplify measurable amounts of *fis* gene sequences from the one or more other microorganism(s) further comprised within the sample.

8. The method of claim 7, wherein the suitable gene amplification technique is PCR (preferably real-time PCR) and wherein the PCR primers are constructed in a way so the PCR primers specifically amplify the analyzed *Legionella pneumophila fis* (factor for inversion stimulation) gene or mRNA expressed from the gene and do not amplify measurable amounts of *fis* gene sequences from the one or more other microorganism(s) further comprised within the sample.

9. The method of claim 8, wherein the PCR primers are selected from the group of PCR primers consisting of:
SEQ ID NO 7 (termed Fis41 F): 5'- CAC TAG CCG AAA GCG TGA CTC -3'; and
SEQ ID NO 8 (termed Fis171 R): 5' ATG TTC CAT TAC TGC ACG AAA TAG AG -3'.

10. The method of claims 8 or 9, wherein the PCR technique is Reverse Transcriptase (RT) PCR and there is detected for presence of viable *Legionella pneumophila* in the sample by specifically amplify mRNA expressed from the analyzed *Legionella pneumophila fis* (factor for inversion stimulation) gene.

## Patentansprüche

1. Verfahren zum spezifischen Nachweis des Vorhandenseins von *Legionella pneumophila* in einer Probe, die im Verdacht steht, *L. pneumophila* zu enthalten, und die weiterhin einen anderen Mikroorganismus oder mehrere andere Mikroorganismen enthält, **dadurch gekennzeichnet, dass**
(i): die Probe auf das Vorhandensein eines *Legionella pneumophila-fis* (factor for inversion stimulation)-Gens hin untersucht wird; und
(ii) die in der Probe vorhandene *Legionella* pneumophila-fis-Gen-Menge ausgewertet wird und, wenn die Probe das *Legionella pneumophila-fis-*Gen enthält, dies ein Beweis für das Vorhandensein von *Legionella pneumophila* in der Probe ist;
wobei das *Legionella pneumophila-fis-*Gen ein *fis*-Gen ist, das ausgewählt wird aus der Gruppe von *fis*-Genen bestehend aus:
(a) einem *fis*-Gen mit einer DNA-Sequenz, die zu mindestens 95% mit der DNA-Sequenz übereinstimmt, die in den Positionen 1-282 der SEQ ID NO 1 ("*fis1*" genannt) dargestellt wird;
(a1) einem *fis*-Gen, das für ein Polypeptid kodiert, das zu mindestens 95% mit dem Polypeptid übereinstimmt, das in den Positionen 1-93 der SEQ ID NO 2 ("FIS1" genannt) dargestellt wird;
(b) einem *fis*-Gen mit einer DNA-Sequenz, die zu mindestens 95% mit der DNA-Sequenz übereinstimmt, die in den Positionen 1-297 der SEQ ID NO 3 ("*fis2*" genannt) dargestellt wird;
(b1) einem *fis*-Gen, das für ein Polypeptid kodiert, das zu mindestens 95% mit dem Polypeptid übereinstimmt, das in den Positionen 1-98 der SEQ ID NO 4 ("FIS2" genannt) dargestellt wird;
(c) einem *fis*-Gen mit einer DNA-Sequenz, die zu mindestens 95% mit der DNA-Sequenz übereinstimmt, die in den Positionen 1-288 der SEQ ID NO 5 ("*fis*3" genannt) dargestellt wird;
(c1) einem *fis*-Gen, das für ein Polypeptid kodiert, das zu mindestens 95% mit dem Polypeptid übereinstimmt, das in den Positionen 1-95 der SEQ ID NO 6 ("FIS3" genannt) dargestellt wird;

2. Verfahren nach Anspruch 1, bei dem der eine andere oder die verschiedenen anderen in der Probe enthaltene/n Mikroorganismus/-men ein Mikroorganismus oder mehrere Mikroorganismen ist/sind, der/die aus der Gruppe ausgewählt wird/werden, die aus *anderen Legionella-* Spezies als *L. pneumophila, E. coli, Salmonella, Shigella, Enterobacter, Micrococcus, Bacillus, Staphylococcus, Pseudomonas, Serratia, Proteus, Enterococcus, Arthrobacter* und *Listeria* besteht.

3. Verfahren nach Anspruch 1, bei dem der eine andere oder die verschiedenen anderen in der Probe enthaltene/n Mikroorganismus/-men eine oder mehrere andere von *L. pneumophila* verschiedene Legionella-Spezies ist/sind, wie z.B. eine oder mehrere Legionella-Spezies, die aus der Gruppe ausgewählt wird/werden, die aus *L. gormanii, L. longbeacheae, L. anisa, L. oakridgensis, L. fairfieldensis, L. feelei, L. dumofii, L. micdadei, L. jordanis, L. wadsworthii* und *L. bozemanii* besteht.

4. Verfahren nach einem der vorstehenden Ansprüche, wobei das Verfahren ein Nachweisverfahren für wachstumsfähige *Legionella pneumophila* in einer Probe ist, das **dadurch gekennzeichnet ist, dass**
(i): die Probe auf das Vorhandensein von mRNA, die von einem *Legionella pneumophila-fis* (factor for inversion stimulation)-Gen exprimiert wurde, hin untersucht wird; und
(ii) die vom *Legionella pneumophila-fis-*Gen exprimierte in der Probe vorhandene mRNA-Menge ausgewertet wird und, wenn die Probe die mRNA vom *Legionella pneumophila-fis-*Gen enthält, dies ein Beweis für das Vorhandensein wachstumsfähiger *Legionella pneumophila* in der Probe ist.

5. Verfahren nach einem der vorstehenden Ansprüche, bei dem die Probe eine Umweltprobe ist, die einer wässrigen Umgebung entnommen wird, wobei die wässrige Umgebung vorzugsweise an einem relevanten Ort wie z.B. in einem Gebäude, in einem Kühlturm, einem häuslichen Trinkwasserverteilungssystem oder im Grundwasser gelegen ist.

6. Verfahren nach einem der vorstehenden Ansprüche, bei dem das *Legionella pneumophila-fis-*Gen ein *fis*-Gen ist, das ausgewählt wird aus einer Gruppe von *fis-*Genen bestehend aus:
(a) einem *fis*-Gen mit einer DNA-Sequenz, die zu mindestens 95% mit der DNA-Sequenz übereinstimmt, die in den Positionen 1-282 der SEQ ID NO 1 ("*fis1*" genannt) dargestellt wird;
(a1) einem *fis*-Gen, das für ein Polypeptid kodiert, das zu mindestens 95% mit dem Polypeptid übereinstimmt, das in den Positionen 1-93 der SEQ ID NO 2 ("FIS1" genannt) dargestellt wird;
oder bevorzugter, bei dem das *Legionella pneumophila-fis-*Gen ein *fis*-Gen ist, das ausgewählt wird aus einer Gruppe von *fis*-Genen bestehend aus:
(a) einem *fis*-Gen mit einer DNA-Sequenz, die mit der DNA-Sequenz übereinstimmt, die in den Positionen 1-282 der SEQ ID NO 1 ("fis1" genannt) dargestellt wird; und
(a1) einem *fis*-Gen, das für ein Polypeptid kodiert, das mit dem Polypeptid übereinstimmt, das in den Positionen 1-93 der SEQ ID NO 2 ("FIS1" genannt) dargestellt wird;

7. Verfahren nach einem der vorstehenden Ansprüche, bei dem die Analyse zur Feststellung des Vorhandenseins eines *Legionella pneumophila-fis* (factor for inversion stimulation)-Gens oder einer vom *fis*-Gen in Übereinstimmung mit Schritt (i) des Verfahrens exprimierten mRNA mittels einer geeigneten Genamplifikationsmethode [z.B. Polymerase-Kettenreaktion (PCR), Ligase-Kettenreaktion (LCR), NASBA (Nukleinsäuresequenz-basierte Amplifikation) oder Strangverdrängungsamplifikation (SDA)] zur Amplifikation der relevanten Gene oder vom Gen exprimierten mRNA vorgenommen wird, und bei dem die Amplifikationsmethode so ausgeführt wird, dass das analysierte *Legionella pneumophila-fis* (factor for inversion stimulation)-Gen oder die vom Gen exprimierte mRNA spezifisch vervielfältigt werden kann und keine messbaren Mengen von *fis*-Gensequenzen des einen oder der verschiedenen anderen ebenfalls in der Probe enthaltenen Mikroorganismus/-men vervielfältigt werden.

8. Verfahren nach Anspruch 7, bei dem die geeignete Genamplifikationsmethode PCR (bevorzugt Real-time-PCR) ist, und bei dem die PCR-Primer so gestaltet sind, dass die PCR-Primer das analysierte *Legionella pneumophila-fis* (factor for inversion stimulation)-Gen oder die vom Gen exprimierte mRNA spezifisch vervielfältigen und keine messbaren Mengen von fis-Gensequenzen des einen oder der verschiedenen anderen ebenfalls in der Probe enthaltenen Mikroorganismus/-men vervielfältigen.

9. Verfahren nach Anspruch 8, bei dem die PCR-Primer ausgewählt werden aus der Gruppe von PCR-Primern bestehend aus:
SEQ ID NO 7 (Fis41 F genannt): 5'-CAC TAG CCG AAA GCG TGA CTC-3'; und
SEQ ID NO 8 (Fis171 R genannt): 5' ATG TTC CAT TAC TGC ACG AAA TAG AG -3'.

10. Verfahren nach Anspruch 8 oder 9, bei dem die PCR-Methode Reverse Transkriptase (RT)-PCR ist, und das Vorhandensein wachstumsfähiger Legionella Pneumophila in der Probe durch spezifische Amplifikation der vom analysierten *Legionella pneumophila-fis* (factor for inversion stimulation)-Gen exprimierten mRNA nachgewiesen wird.

## Revendications

1. Procédé pour la détection spécifique de la présence de *Legionella pneumophila* dans un échantillon qui est soupçonné de contenir *L. pneumophila* et qui comprend en outre un ou plusieurs autre(s) micro-organisme(s), **caractérisé en ce que**
(i) : l'échantillon est analysé pour identifier la présence d'un gène *fis* (factor for inversion stimulation) de *Legionella pneumophila*; et
(ii) la quantité du gène *fis* de *Legionella pneumophila* présente dans l'échantillon est évaluée et si l'échantillon comprend le gène *fis* de *Legionella pneumophila,* il s'agit d'une preuve que la *Legionella pneumophila* est présente dans l'échantillon ;
dans lequel le gène *fis* de *Legionella pneumophila* est un gène *fis* choisi parmi le groupe des gènes *fis* constitués de :
(a) un gène *fis* comprenant une séquence d'ADN qui est au moins identique à 95 % à la séquence d'ADN montrée dans les positions 1-282 de SEQ ID NO 1 (dénommé « *fis1* ») ;
(a1) un gène *fis* qui code pour un polypeptide qui est au moins identique à 95 % au polypeptide montré dans les positions 1-93 de SEQ ID NO 2 (dénommé « FIS1 ») ;
(b) un gène *fis* comprenant une séquence d'ADN qui est au moins identique à 95 % à la séquence d'ADN montrée dans les positions 1-297 de SEQ ID NO 3 (dénommé *« fils2* ») ;
(b1) un gène *fis* qui code pour un polypeptide qui est au moins identique à 95 % au polypeptide montré dans les positions 1-98 de SEQ ID NO 4 (dénommé « FIS2 ») ;
(c) un gène *fis* comprenant une séquence d'ADN qui est au moins identique à 95 % à la séquence d'ADN montrée dans les positions 1-288 de SEQ ID NO 5 (dénommé *« fis3* ») ; et
(c1) un gène *fis* qui code pour un polypeptide qui est au moins identique à 95 % au polypeptide montré dans les positions 1-95 de SEQ ID NO 6 (dénommé « FIS3 ») ;

2. Procédé selon la revendication 1, dans lequel l'un ou les plusieurs autre(s) micro-organisme(s) compris au sein de l'échantillon sont un ou plusieurs micro-organisme(s) choisi(s) dans le groupe constitué d'autres espèces de *Legionella* que *L. pneumophila, E. coli, Salmonella, Shigella, Enterobacter, Micrococcus, Bacillus, Staphylococcus, Pseudomonas, Serratia, Proteus, Enterococcus, Arthrobacter* et *Listeria,*

3. Procédé selon la revendication 1, dans lequel l'un ou les plusieurs autre(s) micro-organisme(s) compris au sein de l'échantillon est/sont une ou plusieurs autre(s) espèces de *Legionella* que *L. pneumophila,* telles qu'en particulier une ou plusieurs espèce(s) de *Legionella* choisie(s) dans le groupe constitué de *L gormanii, L. longbeacheae, L. anisa, L. oakridgensis, L. fairfieldensis, L, feelei, L. dumofii, L. micdadei, L. jordanis, L. wadsworthii* et *L. bozemanii.*

4. Procédé selon l'une quelconque des revendications précédentes, où le procédé est un procédé pour la détection de *Legionella pneumophila* viable dans un échantillon,
**caractérisé en ce que**
(i) : l'échantillon est analysé pour identifier la présence d'ARNm exprimé à partir d'un gène *fis* (factor for inversion stimulation) de *Legionella pneumophila*; et
(ii) la quantité de l'ARNm exprimé à partir du gène *fis* de *Legionella pneumophila* présent dans l'échantillon est évaluée et si l'échantillon comprend l'ARNm du gène *fis* de *Legionella pneumophila,* il s'agit d'une preuve que de la *Legionella pneumophila* viable est présente dans l'échantillon.

5. Procédé selon l'une quelconque des revendications précédentes, dans lequel l'échantillon est un échantillon environnemental obtenu à partir d'un environnement aquatique, de préférence dans lequel l'environnement aquatique est situé dans un endroit représentatif tel qu'un bâtiment, une tour de refroidissement, un système de distribution d'eau potable domestique ou de l'eau souterraine.

6. Procédé selon l'une quelconque des revendications précédentes, dans lequel le gène *fis* de *Legionella pneumophila* est un gène *fis* choisi dans un groupe de gènes *fis* constitués de :
(a) un gène *fis* comprenant une séquence d'ADN qui est au moins identique à 95 % à la séquence d'ADN montrée dans les positions 1-282 de SEQ ID NO 1 (dénommé « *fis1* »); et
(a1) un gène *fis* qui code pour un polypeptide qui est au moins identique à 95 % au polypeptide montré dans les positions 1-93 de SEQ ID NO 2 (dénommé « FIS1 ») ;
ou plus préférablement, dans lequel le gène *fis* de *Legionella pneumophila* est un gène *fis* choisi parmi le groupe des gènes *fis* constitués de :
(a) un gène *fis* comprenant une séquence d'ADN qui est identique à la séquence d'ADN montrée dans les positions 1-282 de SEQ ID NO 1 (dénommé « *fis1* ») ; et
(a1) un gène *fis* qui code pour un polypeptide qui est identique au polypeptide montré dans les positions 1-93 de SEQ ID NO 2 (dénommé « FIS1 ») ;

7. Procédé selon l'une quelconque des revendications précédentes, dans lequel l'analyse pour identifier la présence d'un gène *fis* (factor for inversion stimulation) de *Legionella pneumophila* ou d'ARNm exprimé à partir du gène *fis* conformément à l'étape (i) du procédé, est faite par une technique appropriée d'amplification génique [par exemple, réaction d'amplification en chaîne par polymérase (PCR), réaction en chaîne de ligase (LCR), NASBA (amplification basée sur la séquence d'acides nucléiques) ou amplification par déplacement de brin (SDA)] de façon à amplifier le gène concerné ou l'ARNm exprimé à partir du gène et dans lequel la technique d'amplification est effectuée d'une façon dans laquelle elle est capable d'amplifier spécifiquement le gène *fis* (factor for inversion stimulation) de *Legionella pneumophila* analysé ou l'ARNm exprimé à partir du gène et de ne pas amplifier des quantités mesurables des séquences de gènes *fis* provenant de l'un ou des plusieurs autre(s) micro-organisme(s) compris en outre au sein de l'échantillon.

8. Procédé selon la revendication 7, dans lequel la technique appropriée d'amplification génique est la PCR (de préférence, PCR en temps réel) et dans lequel les amorces de PCR sont construites d'une façon telle que les amorces de PCR amplifient spécifiquement le gène *fis* (factor for inversion stimulation) de *Legionella pneumophila* analysé ou l'ARNm exprimé à partir du gène et n'amplifient pas des quantités mesurables des séquences de gènes *fis* provenant de l'un ou des plusieurs autre(s) micro-organisme(s) compris en outre au sein de l'échantillon.

9. Procédé selon la revendication 8, dans lequel les amorces de PCR sont choisies parmi le groupe d'amorces de PCR constituées de :
SEQ ID NO 7 (dénommée Fis41 F) : 5'-CAC TAG CCG AAA GCG TGA CTC -3' ; et
SEQ ID NO 8 (dénommée Fis171 R) : 5' ATG TTC CAT TAC TGC ACG AAA TAG AG - 3'.

10. Procédé selon les revendications 8 ou 9, dans lequel la technique de PCR est une PCR par transcriptase inverse (RT) et on y détecte la présence de *Legionella pneumophila* viable dans l'échantillon en amplifiant spécifiquement l'ARNm exprimé à partir du gène *fis* (factor for inversion stimulation) de *Legionella pneumophila* analysé.
